# EUROPÄISCHE PATENTSCHRIFT

(11) **EP 1 733 024 B1**
(45) Veröffentlichungstag und Bekanntmachung des Hinweises auf die Patenterteilung: **19.11.2008**
(21) Anmeldenummer: 05729063.7
(22) Anmeldetag: 01.04.2005
(51) Int. Cl.: C12M 3/04, C12N 5/06, C12N 5/00

(54) **VERFAHREN ZUR HERSTELLUNG EINER EPITHELZELLEN ENTHALTENDEN ZELLZUSAMMENSETZUNG**
METHOD FOR THE PRODUCTION OF A CELL COMPOSITION CONTAINING EPITHELIAL CELLS
PROCEDE DE PREPARATION D'UNE COMPOSITION CONTENANT DES CELLULES EPITHELIALES

(30) Priorität: 08.04.2004 DE 102004017476
(43) Veröffentlichungstag der Anmeldung: 20.12.2006
(73) Patentinhaber: Fraunhofer-Gesellschaft zur Förderung der angewandten Forschung e.V., 80686 München (DE)
(72) Erfinder: KRUSE, Charli, 23923 Herrnburg (DE); FUHR, Günter, 13187 Berlin (DE); WEDEL, Thilo, 22552 Lübeck (DE)
(74) Vertreter: Hertz, Oliver
(86) Internationale Anmeldenummer: PCT/EP2005/003430
(87) Internationale Veröffentlichungsnummer: WO 2005/097974

(56) Entgegenhaltungen:
- EP-A- 1 636 347
- WO-A-00/78929
- US-A- 5 122 470
- US-A- 5 190 878
- US-A1- 2003 148 510
- US-A1- 2003 165 484
- RAMIYA VIJAYAKUMAR K ET AL: "Reversal of insulin-dependent diabetes using islets generated in vitro from pancreatic stem cells" NATURE MEDICINE, NATURE AMERICA, NEW YORK, US, Bd. 6, Nr. 3, März 2000 (2000-03), Seiten 278-282, XP000864764 ISSN: 1078-8956
- TOKORO TAKAMASA ET AL: "Differentiation of acinar cells into acinoductular cells in regenerating rat pancreas." PANCREATOLOGY : OFFICIAL JOURNAL OF THE INTERNATIONAL ASSOCIATION OF PANCREATOLOGY (IAP) ... [ET AL.]. 2003, Bd. 3, Nr. 6, 2003, Seiten 487-496, XP008059087 ISSN: 1424-3903
- KRUSE C ET AL: "Pluripotency of adult stem cells derived from human and rat pancreas" APPL PHYS A; APPLIED PHYSICS A: MATERIALS SCIENCE AND PROCESSING NOVEMBER 2004, Bd. 79, Nr. 7, November 2004 (2004-11), Seiten 1617-1624, XP002331149
- WEISSMAN I L ET AL: "Stem and progenitor cells: origins, phenotypes, lineage commitments, and transdifferentiations." ANNUAL REVIEW OF CELL AND DEVELOPMENTAL BIOLOGY. 2001, Bd. 17, 2001, Seiten 387-403, XP002366258 ISSN: 1081-0706
- ANDERSON N L AND ANDERSON N G: "The human plasma proteome. History, character, and diagnostic prospects" MOLECULAR & CELLULAR PROTEOMICS, ASBBM, BIRMINGHAM, AL, US, Bd. 1, Nr. 11, 2002, Seiten 845-867, XP002986053 ISSN: 1535-9476
- LAKSHMANAN J: "Nerve growth factor levels in mouse serum: variations due to stress." NEUROCHEMICAL RESEARCH. APR 1987, Bd. 12, Nr. 4, April 1987 (1987-04), Seiten 393-397, XP008059178 ISSN: 0364-3190
- TAI M -H ET AL: "Role of pancreatic stem cells in the emergence of pancreatic stellate cells and fibroblast-like cells in chronic pancreatitis." PANCREAS, Bd. 27, Nr. 4, November 2003 (2003-11), Seiten 413-414, XP008059097 & MEETING OF THE AMERICAN PANCREATIC ASSOCIATION; CHICAGO, IL, USA; NOVEMBER 06-07, 2003 ISSN: 0885-3177
- GAO RU ET AL: "Characterization of endocrine progenitor cells and critical factors for their differentiation in human adult pancreatic cell culture." DIABETES. AUG 2003, Bd. 52, Nr. 8, August 2003 (2003-08), Seiten 2007-2015, XP002371115 ISSN: 0012-1797

## Beschreibung

Die Erfindung betrifft Verfahren zur Bildung von Epithelzellen, Verfahren zur Kultivierung von Epithelzellen und Kultivierungseinrichtungen für Epithelzellen.

Das Epithel-Gewebe ist allgemein ein Gewebe aus Epithelzellen, die auf allen äußeren und inneren Oberflächen mehrzelliger Organismen Deckschichten bilden und einerseits mechanische Schutz- und Abdeckfunktionen besitzen und andererseits einen Stoffaustausch zwischen dem abgedeckten Gewebe und der Umgebung beeinflussen. Epithel-Gewebe ist bei Wirbeltieren in der Regel mehrschichtig und ggf. aus verschiedenen Epithelzell-Typen aufgebaut. Äußere Epithelzellen werden durch mechanischen Abtrag oder Absterben entfernt, während von dem inneren Gewebe aus einer teilungsaktiven Basalschicht Epithelzellen nachgebildet werden.

In der Biotechnologie und Medizin, insbesondere der Transplantationsmedizin, besteht ein starkes Interesse an Epithelzellen, bspw. um einzelne Zellen oder Zellkulturen zu umhüllen, Gewebe in einem Organismus abzudecken oder Lücken in einem Gewebe auszufüllen, die ggf. durch eine Verletzung oder eine Operation entstanden sind. Beim sog. Tissue Engineering, bei dem Zellen bestimmter Herkunft zu Gewebe mit einer bestimmten Funktion und/oder Form zusammengefügt werden, besteht ein besonderes Interesse an Epithelzellen, da diese in einem künstlich hergestellten Gewebe aus biologischen Zellen insbesondere eine Träger- oder Matrixfunktion übernehmen können.

Probleme bei der bisherigen Verwendung von Epithelzellen ergeben sich daraus, dass die natürliche Erzeugung im Organismus in der Regel auf die genannte Basalschicht beschränkt ist, die aber bspw. beim Tissue Engineering nicht zur Verfügung steht. Des Weiteren kann es bei der Bildung eines Zellpräparats mit heterologen Epithelzellen zu Abstoßungsreaktionen nach einer Transplantation in einem Organismus kommen.

Es ist bekannt, dass aufgrund der pluripotenten Differenzierungsfähigkeit embryonaler Stammzellen grundsätzlich auch Epithelzellen durch Differenzierung aus embryonalen Stammzellen hervorgehen können. Die Bildung von Epithelzellen aus embryonalen Stammzellen unterliegt jedoch aus ethischen Gründen und wegen der begrenzten Verfügbarkeit der embryonalen Stammzellen Beschränkungen und ist bisher experimentell noch nicht befriedigend gelungen.

Ramiya et al. (Nature Medicine, Nature America, New York, US., Bd. 6, Nr. 3, März 2000, Seiten 278-282) offenbaren die Gewinnung und Kultivierung von epithelialen Zellen aus Gängen der Inseln des Pankreas und die Verwendung von daraus entstandenen adulten Stammzellen zur Erzeugung spheroider Zellaggregate, die endokrine α-, β- und γ-Inselzellen umfassen, durch Differenzierung. Diese Veröffentlichung offenbart keine Differenzierung der Stammzellen zu Epithelzellen an einer Grenzfläche zwischen flüssigen und gasförmigen Medien.

Die Aufgabe der Erfindung ist es, ein verbessertes Verfahren zur Bildung von Epithelzellen bereitzustellen, mit dem die Nachteile der herkömmlichen Verfahren überwunden werden und das insbesondere ermöglicht, die Beschränkungen durch die Gewinnung von Epithelzellen aus Basalschichten oder aus embryonalen Stammzellen zu überwinden. Die Aufgabe der Erfindung ist es auch, verbesserte Zellzusammensetzungen bereitzustellen, die mit geringem Aufwand herstellbar sind und einen erweiterten Anwendungsbereich besitzen. Eine weitere Aufgabe der Erfindung ist es, eine Kultivierungseinrichtung zur Bildung von Epithelzellen bereitzustellen.

Diese Aufgaben werden durch Verfahren mit den Merkmalen des Anspruchs 1 und Kultivierungseinrichtungen mit den Merkmalen der Ansprüche 21 und 22 gelöst. Vorteilhafte Ausführungsformen und Anwendungen der Erfindung ergeben sich aus den abhängigen Ansprüchen.

Verfahrensbezogen wird die oben genannte Ausführungsform durch eine Aggregation von Stammzellen, die aus differenziertem exokrinen Drüsengewebe eines Organismus isoliert wurden, zu dreidimensionalen Zellverbänden und ein anschließendes Differenzieren zumindest eines Teils der Zellverbände zu Epithelzellen gelöst. Die Zellverbände werden im Folgenden auch als organoide Körper bezeichnet. Ein besonderer Vorteil dieses Verfahrens besteht in der kompletten Abkopplung der Bildung von Epithelzellen von der herkömmlichen Verwendung von Basalschichten oder embryonalen Stammzellen.

Die Erfinder haben festgestellt, dass die aus den exokrinem Drüsengewebe isolierten Stammzellen pluripotent sind und eine hohe Teilungsfähigkeit und ein starkes Wachstum zeigen. Damit besitzen sie ein Potential, Ausgangsmaterial für die Zelldifferenzierung hin zu Epithelzellen in großem Umfang bereitzustellen.

Das erfindungsgemäß verwendete exokrine Drüsengewebe kann von einem erwachsenen Organismus, juvenilen Organismus oder nicht-humanen fötalen Organismus, vorzugsweise einem postnatalen Organismus, stammen. Der Begriff "adult", wie in der vorliegenden Anmeldung verwendet, bezieht sich somit auf das Entwicklungsstadium des Ausgangsgewebes und nicht auf dasjenige des Donororganismus, aus dem das Gewebe stammt. "Adulte" Stammzellen sind nicht-embryonale Stammzellen.

Vorzugsweise wird das exokrine Drüsengewebe aus einer Speicheldrüse, Tränendrüse, Talgdrüse, Schweißdrüse, aus Drüsen des Genitaltrakts, einschließlich Prostata, oder aus gastrointestinalem Gewebe, einschließlich Pankreas, oder sekretorischem Gewebe der Leber isoliert. In einer stark bevorzugten Ausführungsform handelt es sich dabei um acinäres Gewebe. Ganz besonders bevorzugt stammt das acinäre Gewebe aus dem Pankreas, der Ohrspeicheldrüse oder Unterkieferspeicheldrüse.

Ein wesentlicher Vorteil des erfindungsgemäßen Verfahrens besteht darin, dass die Stammzellen aus lebenden Donororganismen, z. B. aus Speicheldrüsen gewonnen werden können, ohne dass der Donororganismus wesentlich beeinträchtigt wird. Dies ermöglicht die Bildung autologer Epithelzellen, mit denen bei Transplantation in den Donororganismus die oben genannten Abstoßungsreaktionen vermieden werden können.

Allgemein kann erfindungsgemäß die Epithelzusammensetzung unmittelbar aus dem organoiden Körper gebildet werden. Gemäß einer bevorzugten Ausführungsform der Erfindung erfolgt jedoch zunächst ein Wachstum der organoiden Körper z. B. in einer Suspensionskultur oder einer Adhäsionskultur unter Nährstoffversorgung zu größeren Körpern, die im Folgenden als Gewebekörper bezeichnet werden. Die Erfinder haben festgestellt, dass die aus den exokrinen Drüsen isolierten Stammzellen organoide Körper bilden, die bei der Nährstoffversorgung ein starkes Wachstum bis zu Durchmessern von einigen Millimetern oder darüber zeigen. Die Präparation der Epithelzellen aus den Gewebekörpern besitzt den Vorteil einer größeren Ausbeute und Effektivität.

Allgemein kann das Differenzieren des organoiden Körpers oder des Gewebekörpers durch den Zusatz eines Differenzierungsfaktors, wie z. B. eines molekularen Wachstumsfaktors oder differenzierter Epithelzellen, in das Kultivierungsmedium augelöst werden, um Epithelzellen zu bilden und ggf. im Kultivierungsmedium wachsen zu lassen. Im Verfahren der Erfindung erfolgt jedoch das Differenzieren von Zellen des organoiden Körpers zu Epithelzellen durch ein Kultivieren an einer Grenzfläche zwischen einem flüssigen und gasförmigen Medium. Die Erfinder haben festgestellt, dass die Epithelzellen sich spontan bilden, sobald ein organoider Körper oder ein Gewebekörper aus dem flüssigen Kultivierungsmedium in eine gasförmige Umgebung, z. B. in die Umgebungsluft herausragt oder herauswächst. In diesem Fall kann auf Wachstumsfaktoren oder stimulierende Zellen im Kultivierungsmedium verzichtet werden, das Kultivierungsmedium kann frei von Wachstumsfaktoren oder stimulierenden Zellen sein. Der organoide Körper, der der gasförmigen Umgebung ausgesetzt wird, kann aus wenigen Stammzellen bis hin zu einer einzelnen Zelle bestehen.

Für eine gezielte Differenzierung kann es von Vorteil sein, wenn gemäß einer weiteren bevorzugten Ausführungsform der Erfindung beim Kultivieren der organoiden Körper an der Grenzfläche zwischen den flüssigen und gasförmigen Medien die Flüssigkeitsoberfläche des flüssigen Mediums, z. B. des Kultivierungsmediums relativ zur Oberfläche des organoiden Körpers verschoben wird, so dass dieser im Verlauf des Kultivierungsschrittes zunehmend aus dem flüssigen Medium herausragt.

Diese Verschiebung der Flüssigkeitsoberfläche kann erreicht werden, indem wenigstens einer der folgenden Vorgänge gleichzeitig oder aufeinanderfolgend bereitgestellt wird. Wenn die Flüssigkeitsoberfläche sich durch ein Herauswachsen des organoiden Körpers oder des Gewebekörpers aus dem Kultivierungsmedium verschiebt, ist die Differenzierungsgeschwindigkeit vorteilhafterweise an die Wachstumsgeschwindigkeit des organoiden Körpers angepasst. Wenn die Flüssigkeitsoberfläche des flüssigen Mediums relativ zum organoiden Körper oder Gewebekörper abgesenkt wird, kann die Differenzierungsgeschwindigkeit vorteilhafterweise beeinflusst werden. Wenn die Flüssigkeitsoberfläche schneller abgesenkt wird, als das Gewebe wächst, kann die Differenzierungsgeschwindigkeit vorteilhafterweise sogar erhöht werden. Entsprechendes gilt für ein Anheben eines Kultivierungssubstrats mit dem organoiden Körper aus dem flüssigen Medium.

Besonders bevorzugt wird die Flüssigkeitsoberfläche mit einer Geschwindigkeit relativ zur Oberfläche des organoiden Körpers oder Gewebekörpers verschoben, die an eine Umwandlungsgeschwindigkeit beim Differenzieren der aggregierten Zellen des organoiden Körpers oder Gewebekörpers zu Epithelzellen angepasst ist. Da die Zellen innerhalb des organoiden Körpers durch eine inherente Umordnung der Adhäsionskontakte zu ihrer jeweiligen Umgebung beweglich sind, wird vorzugsweise eine Geschwindigkeit der Flüssigkeitsoberfläche relativ zum organoiden Körper eingestellt, die kleiner oder gleich der Geschwindigkeit der Zellbewegung entsprechend der molekularen Bindungsgeschwindigkeit der Zellkontakte ist. Damit können sich die Zellen optimal an die Differenzierungsbedingungen im gasförmigen Medium anpassen. Die Relativgeschwindigkeit ist bspw. kleiner oder gleich 1000 µm/h.

Besondere Vorteile des erfindungsgemäßen Verfahrens können sich ergeben, wenn der Differenzierungsvorgang unmittelbar mit einer Formgebung verbunden wird. Damit können bestimmte Gewebestücke hergestellt werden, deren Form an die jeweilige Anwendung z. B. bei Transplantationsaufgaben oder beim Tissue-Engineering angepasst sind. Hierzu erfolgt das Differenzieren des organoiden Körpers oder des Gewebekörpers vorzugsweise in unmittelbarer Nachbarschaft an eine Prägeoberfläche mit einer vorbestimmten Form, der die differenzierten Epithelzellen folgen.

Zur massenhaften Bildung differenzierter Epithelzellen ist gemäß einer weiteren Ausführungsform der Erfindung vorgesehen, dass die differenzierten Epithelzellen von den organoiden Körpern oder Gewebekörpern getrennt und gesondert vermehrt werden. Diese Vermehrung erfolgt vorzugsweise auf einem flüssigkeitsdurchlässigen Kultivierungssubstrat, so dass eine allseitige Nährstoffversorgung der Epithelzellen gewährleistet wird. Als Kultivierungssubstrat wird vorzugsweise ein Kollagenvlies oder ein textiles Gewebe verwendet. Diese Materialien können biokompatibel gewählt werden und können Bestandteil der Epithelzellzusammensetzung auch bei deren Anwendung bleiben. Gemäß bevorzugten Varianten erfolgt die Vermehrung der Epithelzellen auf modifizierten Oberflächen des Kultivierungssubstrats, wie z. B. auf fibrin- oder chitinhaltigen Oberflächen.

Wenn die Vermehrung der Epithelzellen auf einem deformierbaren Kultivierungssubstrat erfolgt, können sich weiteren Vorteile für die Anpassung der Form der Epithelzellzusammensetzung an die gewünschte Anwendung ergeben. Alternativ oder zusätzlich kann die Kultur der vermehrten Epithelzellen einer Deformation, z. B. mit einer Stempel- oder Prägeeinrichtung unterzogen werden.

Die zur Bildung der Epithelzellen verwendeten Stammzellen werden vorzugsweise aus einer exokrinen Drüse eines Wirbeltieres, insbesondere eines Fisches, eines Vogels oder eines Säugetieres, insbesondere des Menschen isoliert. Bei der Isolation humaner Stammzellen erfolgt die Isolation aus juvenilen oder adulten Menschen.

Wenn beim Kultivieren an der Grenzfläche die Flüssigkeitsoberfläche des Kultivierungsmediums relativ zur Oberfläche des organoiden Körpers oder des Gewebekörpers wiederholt, zum Beispiel periodisch oder als Schwingung mit einer Richtungsumkehr verschoben wird und der organoide Körper oder der Gewebekörper entsprechend wiederholt mit dem Kultivierungsmedium überspült werden oder freiliegen, können sich Vorteile für eine beschleunigte Bildung der Epithelzellen und deren Versorgung ergeben.

Eine Zellzusammensetzung, die mindestens eine Epithelzelle enthält oder komplett aus Epithelzellen besteht, und nach dem erfindungsgemäßen Verfahren hergestellt ist, stellt ein ziel der Erfindung dar. Bevorzugte Anwendungen der Zellzusammensetzung bestehen bei der Verwendung als Arzneimittel, insbesondere als autologes Arzneimittel, wie z. B. als Transplantat oder Teil eines Transplantats, oder als Material für Tissue-Engineering-Anwendungen. Eine bevorzugte Anwendung der erfindungsgemäß hergestellten Epithelzellzusammensetzung besteht in der synthetischen Präparation biologischen Gewebes.

Vorrichtungsbezogen wird die oben genannte Aufgabe durch die Bereitstellung einer Kultivierungseinrichtung mit der Merkmalen der Ansprüche 21 und 22 gelöst, die ein Kultivierungssubstrat zur Aufnahme von mindestens einem organoiden Körper oder Gewebekörper in einem Kultivierungsgefäß zur Aufnahme eines flüssigen Kultivierungsmediums umfasst, wobei eine Steuereinrichtung zur Einstellung der Flüssigkeitsoberfläche der Kultivierungsflüssigkeit im Kultivierungsgefäß relativ zur Oberfläche des Kultivierungssubstrats vorgesehen ist. Gemäß einer ersten Ausführungsform der Erfindung enthält die Steuereinrichtung eine Stelleinrichtung zur Positionierung und/oder Verschiebung des Kultivierungssubstrats in einer vorbestimmten Höhe im Kultivierungsgefäß. Alternativ oder zusätzlich umfasst die Steuereinrichtung eine Pegelsteuerung zur Einstellung eines Füllstandes der Kultivierungsflüssigkeit im Kultivierungsgefäß.

Gemäß der bevorzugten Ausführungsform der Erfindung nach Anspruch 21 ist die erfindungsgemäße Kultivierungseinrichtung mit einem Prägewerkzeug ausgestattet, das eine Prägeoberfläche zur Formgebung des differenzierten Epithelgewebes besitzt.

Weitere Einzelheiten und Vorteile der Erfindung werden im Folgenden unter Bezug auf die beigefügten Zeichnungen erläutert. Es zeigen:
- Figur 1:: ein Flussdiagramm zur Illustration einer Ausführungsform des erfindungsgemäßen Verfahrens,
- Figur 2:: eine Illustration der Bildung primärer organoider Körper,
- Figur 3:: ein Flussdiagramm zur Illustration weiterer Einzelheiten des erfindungsgemäßen Verfahrens,
- Figur 4:: eine. Darstellung der Bildung sekundärer organoider Körper,
- Figur 5:: eine Photographie eines organoiden Körpers,
- Figur 6:: optische und elektronenmikroskopische Darstellungen von Epithelzellen,
- Figur 7:: ein Flussdiagramm mit weiteren Einzelheiten des erfindungsgemäßen Verfahrens,
- Figur 8:: eine Prinzipdarstellung zur Illustration von Merkmalen einer Kultivierungseinrichtung gemäß der Erfindung,
- Figur 9:: ein Flussdiagramm mit weiteren Einzelheiten des erfindungsgemäßen Verfahrens, und
- Figuren: 10 und 11: schematische Illustrationen von Vorrichtungen zum Tissue-Engineering.

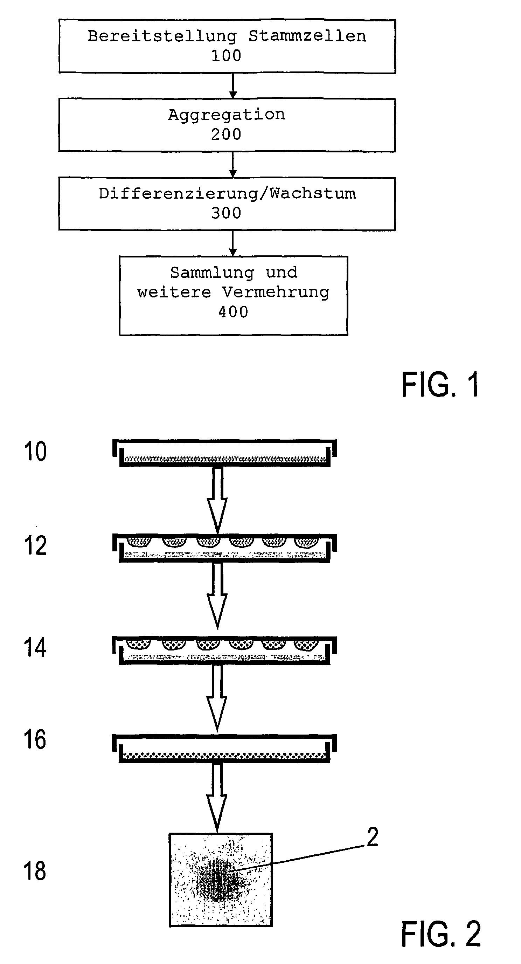

Zur erfindungsgemäßen Erzeugung einer Zusammensetzung aus biologischen Zellen und/oder Gewebe erfolgen gemäß Figur 1 zunächst eine Bereitstellung der von einem Donororganismus isolierten Stammzellen (Schritt 100), anschließend deren Aggregation zu organoiden Körpern (Schritt 200), die einer Differenzierung und/oder einem Wachstum (Schritt 300) und schließlich einer Sammlung und weiteren Bearbeitung, insbesondere Vermehrung (Schritt 400) unterzogen werden. Obwohl die Schritte 100 und 200 im Wesentlichen den erfindungsgemäßen Aggregationsschritt umfassen, während die Schritte 300 und 400 im Wesentlichen den erfindungsgemäßen Differenzierungsschritt repräsentieren und diese beiden Komplexe im Folgenden getrennt erörtert werden, wird betont, dass Teilschritte der Differenzierung bereits im Rahmen der Aggregation erfolgen und umgekehrt Teilschritte der Aggregation erst im Rahmen der Präparation realisiert werden können. So kann bspw. die Differenzierung der organoiden Körper bereits im Rahmen der Aggregation beginnen, oder es können isolierte Stammzellen mit bereits vordifferenzierten organoiden Körpern aggregieren.

Die im Folgenden erläuterten Ausführungsformen der Erfindung beziehen sich beispielhaft auf die Erzeugung der biologischen Materialzusammensetzung aus Stammzellen, die Ratten oder Menschen entnommen wurden. Die Umsetzung der Erfindung ist jedoch nicht auf diese Organismen beschränkt. Vielmehr können die entsprechenden Verfahren an allen Organismen mit differenzierten exokrinen Drüsen mit acinärem Gewebe realisiert werden, so z. B. mit Fischen, die acinäres Gewebe an der Pankreasdrüse besitzen, oder Säugetieren, wie zum Beispiel Rindern oder Schafen.

### (I) Aggregation von Stammzellen aus einer differenzierten exokrinen Drüse eines lebenden Organismus zu organoiden Körpern und/oder Gewebekörpern

Entsprechend dem in Figur 2 dargestellten Schema wird zur Gewinnung der Zellen acinäres Gewebe - bevorzugt einer Speicheldrüse oder der Bauchspeicheldrüse (Pankreas) - mechanisch und enzymatisch zerkleinert in Kultur genommen (Schritt 10 in Figur 2). Entgegen den Angaben von Bachem et al., Gastroenterol. 115:421-432 (1998), und Grosfils et al., Res. Comm. Chem. Pathol. Pharmacol. 79:99-115 (1993), werden keine Gewebeblöcke kultiviert, aus denen Zellen auswachsen sollen, sondern unter der Bedingung, dass die Zellverbände der Acini weitestgehend intakt bleiben, das Gewebe stärker zerkleinert.

Über mehrere Wochen werden diese Zellen und Zellverbände in Kulturgefäßen kultiviert. Alle 2 bi 3 Tage wird das Medium gewechselt, wobei alle differenzierten Zellen entfernt werden. Bei den in Kultur persistierenden Zellen handelt es sich um undifferenzierte Zellen mit uneingeschränkter Teilungsfähigkeit.

Ähnliche Zellen sind unter gleichen Bedingungen aus dem Pankreas isoliert und beschrieben und als eine Art Myofibroblasten bzw. pankreatische Sternzellen bezeichnet worden (Bachem et al., 1998). Im Gegensatz zu den Zellen der vorliegenden Erfindung konnte eine uneingeschränkte Teilungsfähigkeit jedoch nicht beobachtet werden. Weiterhin konnten diese Zellen auch nicht unbegrenzt passagiert werden, ohne an Vitalität zu verlieren.

In einem zweiten Schritt (12) werden ungefähr 400 bis 800 Zellen in je 20 µl Medium in hängenden Tropfen kultiviert. Dazu werden die Tropfen auf Deckel von bakteriologischen Petrischalen gegeben, umgedreht und über die mit Medium gefüllte Petrischale gelegt, so dass die Tropfen nach unten hängen.

Durch diese Art der Kultivierung bilden sich innerhalb von 48h die als organoide Körper bezeichneten Zellaggregate (14), die für ungefähr 6 Tage in eine Suspensionskultur umgesetzt werden (16). Die Teilansicht (18) aus Figur 2 zeigt eine mikroskopische Aufnahme eines solchen organoiden Körpers 2.

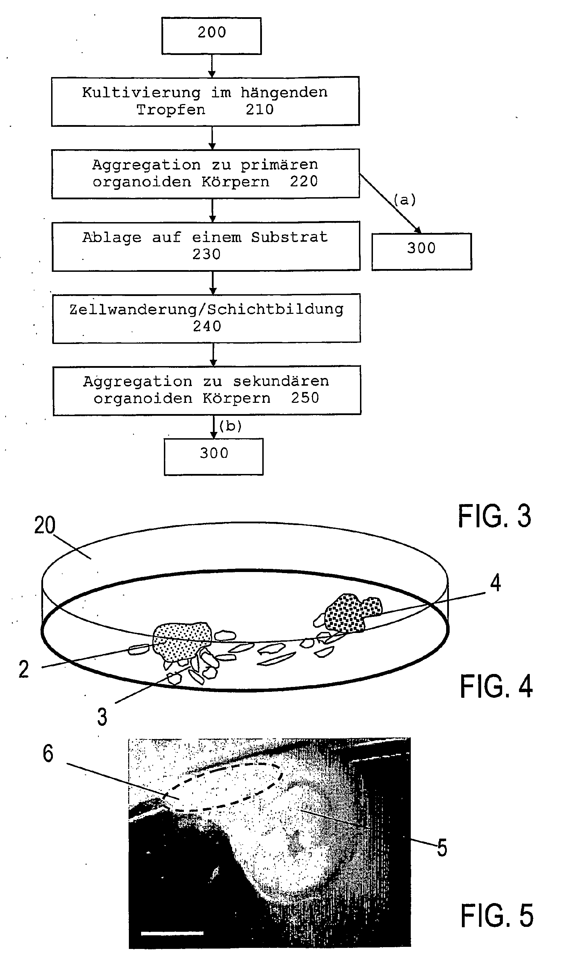

In Figur 3 sind die verschiedenen Möglichkeiten, organoide Körper als Ausgangsmaterialien für die weitere Differenzierung bereitzustellen, mit weiteren Einzelheiten zusammengestellt. Nach der oben beschriebenen Kultivierung der isolierten Stammzellen im hängenden Tropfen (Schritt 210) erfolgt die Aggregation zu den so genannten primären organoiden Körpern (Schritt 220), die direkt der Differenzierung und dem Wachstum (Schritt 300 in Figur 1) unterzogen werden können.

Alternativ erfolgt zunächst die Ablage der primären organoiden Körper auf einem Substrat (Schritt 230) zur Schaffung einer Adhäsionskultur (siehe auch Figur 4). Die Erfinder haben festgestellt, dass die in Suspensionskultur wachsenden primären organoiden Körper in Adhäsionskultur neue organoide Körper bilden können. Auf dem Substrat folgt durch eine Zellwanderung die Bildung einer Monoschicht (Schritt 240) und aus dieser die Aggregation zu den so genannten sekundären organoiden Körpern (Schritt 250). Diese können wiederum direkt der weiteren Differenzierung und/oder dem Wachstum (Schritt 300) unterzogen werden.

Die Bildung der sekundären organoiden Körper ist auch in Figur 4 illustriert. Die primären organoiden Körper 2 bilden auf dem Substrat der Adhäsionskultur, wie z. B. auf dem Boden der Kulturschale 20, durch ein Wandern oder Wachsen von Zellen 3 zunächst eine Monoschicht, aus der dann die sekundäre organoide Körper 4 herauswachsen. Mit der Kultivierung der primären organoiden Körper 2 zu sekundären organoiden Körpern 4 wird eine weitere Vervielfältigung des Biomaterials geschaffen. Aus jedem der primären oder sekundären organoiden Körper 2, 4 können bei weiterer Kultivierung Gewebekörper wachsen.

### Ausführungsbeispiele für die Isolierung und Aggregation von Stammzellen

In den folgenden, nicht-beschränkenden Beispielen wird die Isolierung und Aggregation von Stammzellen näher erläutert.

Die allgemeinen Arbeitsanweisungen, wie sie für Verfahren zur Kultivierung von biologischen Zellen und insbesondere von Säugetierzellen gebräuchlich sind, sind zu beachten. Eine sterile Umgebung, in der das Verfahren durchgeführt werden soll, ist - auch wenn hierzu keine weitere Beschreibung erfolgt - in jedem Fall einzuhalten. Folgende Puffer und Medien wurden verwendet:

| | |
|---|---|
| HEPES-Stammlösung (pH 7,6) | 2,3 83g HEPES auf 100 ml A. *bidest.* |
| HEPES-Eagle-Medium (pH 7,4) | 90 ml Modified Eagle Medium (MEM) |
| | 10 ml HEPES-Stammlösung |
| Isolationsmeditun (pH 7,4) | 32 ml HEPES-Eagle-Medium |
| | 8 ml 5% BSA in A. bidest. |
| | 300 µl 0,1 M CaC12 |
| | 100 µl Trasylol (200.000 KIE) |
| Digestionsmedium (pH 7,4) | 20 ml Isolationsmedium |
| | 4ml Kollagenase (Kollagenase NB 8 von Serva) |
| Inkubationsmedium | Dulbecco's Modified Eagle Medium (DMEM) |
| Nährmedium | Dulbecco's Modified Eagle Medium (DMEM) |
| | DMEM + 4500 mg/l Glucose |
| | + L-glutamin |
| | - Pyruvat |
| | + 20 % FKS (inaktiviert) + 1 ml/100 ml Pen/Strep (10000 E/10000 µg/ml) |
| | oder |
| | DMEM + 10 % Eigenplasma + 1 ml/100 ml Pen/Strep, |
| | vor Gebrauch auf 37°C erwärmen |
| Differenzierungsmedium | 380 ml DMEM |
| | 95 ml 30 min bei 54 °C inaktiviertes FKS |
| | 5 ml Glutamin (GIBCO BRL) |
| | 5 ml (3,5 µl β-Mercaptoethanol auf 5 ml PBS) |
| | 5 ml Non-essential amino acids (GIBCO BRL) |
| | 5 ml Penicillin/Streptomycin (GIBCO BRL) |
| | (10000 E/10000 µg/ml) |

Statt fötalem Kalbserum (FKS) im Nährmedium und Differenzierungsmedium kann gegebenenfalls auch Eigenplasma oder, weniger bevorzugt, Eigenserum des Gewebedonors verwendet werden. Dies ist insbesondere dann von Bedeutung, wenn der Gewebedonor mit dem späteren Empfänger der Stammzellen oder davon abgeleiteten differenzierten Zellen identisch ist. Eine solche autologe Behandlung ist zur Verhinderung einer eventuellen Abstoßungsreaktion bevorzugt.

Das Nährmedium kann als Basismedium statt des verwendeten DMEM-Mediums auch ein anderes.für die Kultivierung von eukaryotischen Zellen, insbesondere Säugerzellen, bekanntes, geeignetes Basismedium enthalten, in dem die differenzierten Zellen absterben und sich die gewünschten Stammzellen vermehren. Auch Isolationsmedium, Inkubationsmedium und Differenzierungsmedium können ein anderes übliches und geeignetes Basismedium enthalten.

Die folgenden Beispiele 1 und 2 beschreiben detailliert zwei Arbeitsprotokolle zur Isolierung und Kultivierung adulter pluripotenter Stammzellen aus acinärem Gewebe des Pankreas. Beispiel 3 beschreibt ein entsprechendes Protokoll für die Isolierung aus acinärem Gewebe der Speicheldrüse.

### BEISPIEL 1

### 1. Präparation des Gewebes und Isolierung der Zellen

In den *Ductus pancreaticus* von 2-3 Jahre alten Ratten werden mittels einer Spritze und einer stumpfen Kanüle bei der Ratte 10 ml Digestionsmedium langsam und luftblasenfrei injiziert. Das gesamte Pankreas wird dadurch aufgeblasen und kann so besser herauspräpariert werden. Das Pankreas wird dann in ein Becherglas überführt und weitere 5 ml Digestionsmedium dazugegeben. Nachdem man das Fettgewebe und Lymphknoten entfernt hat, wird das Gewebe im Becherglas mit einer feinen Schere sehr fein zerkleinert, oben schwimmendes Fettgewebe abgesaugt und die Suspension wird abschließend 1 min mit Carbogen begast (wenn nötig wiederholen) und für 20 min bei 37 °C mit Alufolie bedeckt in einem Schüttler bei 200 Zyklen/min inkubiert. Danach saugt man das Medium vorsichtig ab, zerkleinert erneut mit einer Schere das Gewebe und wäscht die Gewebestücke zweimal mit je 10 ml Isolationsmedium und gibt wieder 5 ml Digestionsmedium zum Gewebe hinzu.

Nach erneutem Begasen mit Carbogen für etwa 1 min und Inkubation für 15 min bei 37 °C in einem Schüttler bei 200 Zyklen/min werden die Gewebestücke durch sukzessives Aufziehen in jeweils einer 10 ml, 5m1, 2 ml und 1 ml Glaspipette zerkleinert und durch einlagiges Filtergewebe gepresst. Die so vereinzelten Zellen werden nun fünfmal in Inkubationsmedium gewaschen (37 °C), mit Carbogen begast und jedes Mal 5 min bei 90 g zentrifugiert. Das zuletzt erhaltene Pellet wird in Inkubationsmedium resuspendiert, begast und auf Gewebekulturschalen verteilt.

### 2. Kultivierung der Zellen

Die Gewebekulturschalen mit den isolierten Zellen werden im Brutschrank bei 37°C und 5 % CO₂ kultiviert. Alle 2-3 Tage wird das Medium gewechselt. Dabei werden alle differenzierten Zellen entfernt.

Am siebenten Tag in Kultur werden die Zellen mit einer Lösung bestehend aus 2 ml PBS, 1 ml Trypsin und 2 ml Inkubationsmedium passagiert. Dabei lösen sich die Zellen vom Boden der Kulturschale. Die Zellsuspension wird 5 Minuten zentrifugiert, der Überstand abgesaugt und die Zellen in 2 ml Inkubationsmedium resuspendiert, auf eine mittlere Zellkulturflasche überführt und 10 ml Inkubationsmedium dazugegeben. Der Medienwechsel erfolgt alle drei Tage.

Am vierzehnten Tag in Kultur werden die Zellen erneut, aber diesmal mit 6 ml PBS, 3 ml Trypsin und 6 ml Inkubationsmedium, passagiert. Die Zellsuspension wird 5 Minuten zentrifugiert, der Überstand abgesaugt und die Zellen in 6 ml Inkubationsmedium resuspendiert, auf 3 mittlere Zellkulturflaschen überführt und jeweils 10 ml Inkubationsmedium dazugegeben.

Die Zellen werden weiter kultiviert und so oft passagiert und ausgesät, bis die Zellen einen semikonfluenten bis konfluenten Zustand erreichen.

### BEISPIEL 2

Pankreas-Acini wurden von männlichen Sprague-Dawley-Ratten (20-300 g) erhalten, die narkotisiert (CO₂) und über die dorsale Aorta ausgeblutet worden waren. Eine Kanüle wurde trasduodenal in den Pankreasgang eingeführt und dem Pankreas wurde von hinten 10 ml Digestionsmedium, enthaltend HEPES-Eagle-Medium (pH 7,4), 0,1 mM HEPES-Puffer (pH, 7,6), 70% (Vol./Vol.) modifiziertes Eagle-Medium, 0,5 % (Vol./Vol.) Trasylol (Bayer AG, Leverkusen, Deutschland), 1 % (Gew./Vol.) Rinderserumalbumin), 2,4 mM CaCl₂ und Kollagenase (0,63 P/mg, Serva, Heidelberg, Deutschland) injiziert.

Vor der Entfernung wurde der Pankreas von anhaftendem Festgewebe, Lymphknoten und Blutgefäßen teilweise befreit. Dann wurde gesundes Pankreasgewebe in Digestionsmedium abgeholt (bei 20 °C, geringerer Stoffwechsel), das Pankreasgewebe mit einer Schere sehr fein zerkleinert, oben schwimmendes Fettgewebe abgesaugt und die Gewebesuspension mit Carbogen (Messer, Krefeld, Deutschland) begast, ohne dass die Düse in das Medium mit den Zellen gelangt (Verringerung von mechanischem Streß) und damit auf pH 7,4 eingestellt. Danach wurde die Suspension in einem 25-ml-Erlenmeyerkolben (mit Alufolie bedeckt) unter konstantem Schütteln (150-200 Zyklen pro Minute) bei 37 °C in 10 ml Digestionsmedium inkubiert. Nach 15-20 Minuten wurde das oben schwimmende Fett und das Medium abgesaugt und das Gewebe wurde erneut zerkleinert und mit Medium ohne Kollagenase gespült (Vorgang mindestens zweimal wiederholen, vorzugsweise solange bis Zellfraktion transparent), worauf Digestionsmedium zugegeben und erneut etwa 1 Minute lang mit Carbogen begast wurde. Es folgte wiederum eine Digestion mit Kollagenase für 15 Minuten bei 37°C im Schüttler unter Verwendung desselben Puffers. Nach der Digestion wurden die Acini durch sukzessives Hochziehen und Ausstossen durch 10 ml-, 5 ml und 2 ml-Glaspipetten mit engen Öffnungen dissoziiert und durch ein einlagiges Nylonsieb (Polymon PES-200/45, Angst & Pfister AG, Zürich, Schweiz) mit einer Maschengröße von etwa 250 µm filtriert. Die Acini wurden zentrifugiert (bei 37°C und 600-800 UpM in einer Beckman-GPR-Zentrifuge, entspricht etwa 90 g) und weiter gereinigt durch Waschen in Inkubationsmedium, enthaltend 24,5 mM HEPES (pH 7,5), 96 mM NaCl, 6 mM KCl, 1 mM MgCl₂, 2,5 mM NaH₂PO₄, 0, mM CaCl₂, 11,5 mM Glucose, 5 mM Natriumpyruvat, 5 mM Natriumglutamat, 5 mM Natriumfumarat, 1 % (Vol./Vol.) modifiziertes Eagle-Medium, 1 % (Gew./Vol.) Rinderserumalbumin, mit Carbogen äquilibriert und auf pH 7,4 eingestellt. Die Waschprozedur (Zentrifugation, Absaugen, Resuspension) wurde fünfmal wiederholt. Soweit nicht anders angegeben, wird bei der obigen Isolierung bei etwa 20 °C gearbeitet.

Die Acini wurden in Inkubationsmedium resuspendiert und bei 37°C in einer angefeuchten Atmosphäre mit 5 % CO₂ kultiviert. Das acinäre Gewebe starb dabei schnell (innerhalb von zwei Tagen) ab und die sterbenden differenzierten Zellen lösten sich dabei von den benachbarten Zellen, ohne diese zu schädigen (schonende Isolierung), die nicht absterbenden Stammzellen sanken zu Boden und hefteten sich an. Die differenzierten Acinizellen sind dazu nicht in der Lage. Das Inkubationsmedium wurde am zweiten oder dritten Tag nach dem Aussäen erstmals gewechselt, wobei ein Großteil der frei schwimmenden Acini und acinären Zellen entfernt wurde. Zu diesem Zeitpunkt hatten sich die ersten Stammzellen bzw. deren Vorläufer am Boden festgesetzt und begannen sich zu teilen. Der Mediumwechsel wurde danach an jedem dritten Tag wiederholt und differenzierte acinäre Pankreaszellen wurden bei jedem Mediumwechsel entfernt.

Am siebenten Tag in Kultur wurden die Zellen mit einer Lösung bestehend aus 2 ml PBS, 1 ml Trypsin (+ 0,05 % EDTA) und 2 ml Inkubationsmedium passagiert. Dabei lösen sich die Zellen vom Boden der Kulturschale. Die Zellsuspension wurde 5 Minuten bei etwa 1000 UpM (Beckmann GPR-Zentrifuge) zentrifugiert, der Überstand abgesaugt und die Zellen in 2 ml Inkubationsmedium resuspendiert, auf eine mittlere Zellkulturflasche überführt und 10 ml Inkubationsmedium dazugegeben.

Am vierzehnten Tag in Kultur wurden die Zellen erneut, aber diesmal mit 6 ml PBS, 3 ml Trypsin/EDTA und 6 ml Inkubationsmedium, passagiert. Die Zellsuspension wird 5 Minuten bei 1000 UpM zentrifugiert, der Überstand abgesaugt und die Zellen in 6 ml Inkubationsmedium resuspendiert, auf 3 mittlere Zellkulturflaschen überführt und jeweils 10 ml Inkubationsmedium dazugegeben.

Am Tag 17 erfolgte ein drittes Passagieren auf insgesamt 6 mittlere Zellkulturflaschen und am Tag 24 ein viertes Passagieren auf insgesamt 12 mittlere Zellkulturflaschen. Spätestens jetzt waren alle primären Zellen bis auf die Stammzellen aus der Zellkultur entfernt.

Die Stammzellen können weiter kultiviert werden und so oft passagiert und ausgesät wie gewünscht. Das Aussäen erfolgt vorzugsweise jeweils in einer Dichte von 2-4 x 10⁵ Zellen/cm² in Inkubationsmedium.

### BEISPIEL 3

Die Isolierung und Kultivierung aus exokrinem Gewebe der Ohrspeicheldrüse eines Menschen erfolgte analog dem Pankreas-Protokoll mit den folgenden Abweichungen:
1. Das exokrine Gewebe der Ohrspeicheldrüse war eine Mischung von acinärem Gewebe und tubulösem Gewebe.
2. Nachdem Speicheldrüsen weniger Proteasen und Amylasen als Pankreas enthalten, ist es möglich, das Speicheldrüsengewebe vor der Aufarbeitung einige Zeit im Kühlschrank bei etwa 4°C aufzubewahren, ohne dass das Gewebe zu sehr geschädigt wird. Im konkreten Beispielsfall betrug die Aufbewahrungszeit 15 h und brachte keine nachteiligen Folgen für die Isolierung der gewünschten Stammzellen mit sich.

Das folgende Beispiel 4 beschreibt detailliert ein Arbeitsprotokoll zur Herstellung von organoiden Körpern.

### BEISPIEL 4

Die undifferenzierten Zellen werden mit einer Lösung aus 10 ml PBS, 4 ml Trypsin, 8 ml Differenzierungsmedium abtrypsiniert und 5 Minuten abzentrifugiert. Das resultierende Pellet wird so in Differenzierungsmedium resuspendiert, dass sich eine Verdünnung von 3000 Zellen je 100 µl Medium einstellt. Anschließend werden die Zellen nochmals gut mit einer 3 ml Pipette suspendiert.

Von bakteriologischen Petrischalen, die vorher mit jeweils 15 ml PBS (37 °C) pro Platte beschichtet worden sind, wird der Deckel abgenommen und umgedreht. Mit Hilfe einer automatischen Pipette werden auf einen Deckel ca. fünfzig 20 µl Tropfen gegeben. Der Deckel wird dann schnell umgedreht und auf die mit Differenzierungsmedium gefüllte Petrischale gegeben, sodass die Tropfen nach unten hängen. Die Petrischalen werden anschließend vorsichtig in den Brutschrank gestellt und für 48 h inkubiert.

Daraufhin werden die in den hängenden Tropfen aggregierten Zellen, die die organoiden Körper bilden, aus jeweils vier Deckeln in je eine bakteriologische Petrischale mit 5 ml Inkubationsmedium mit 20 % FKS überführt und für weitere 96 h kultiviert.

Die organoiden Körper werden nun vorsichtig mit einer Pipette aufgesammelt, und in mit 0,1 % Gelatine beschichtete Zellkulturgefäße mit Differenzierungsmedium überführt. In einer besonders bevorzugten Ausgestaltung des Verfahrens verwendet man als Kulturgefäß mit 0,1 % Gelatine beschichtete 6 cm Petrischalen, in die 4 ml Differenzierungsmedium vorgelegt wurde und die anschließend mit je 6 organoiden Körpern beschickt werden. Ein weiteres bevorzugtes Kulturgefäß sind mit 0,1 % Gelatine beschichtete Chamber Slides, in die 3 ml Differenzierungsmedium vorgelegt wurde und die anschließend mit je 3-8 organoiden Körpern beschickt werden. Daneben können auch 24-Mulden-Mikrotiterplatten verwendet werden, die mit 0,1 % Gelatine beschichtet wurden und in die je 1,5 ml pro Mulde Differenzierungsmedium vorgelegt wurde und anschließend mit je 4 organoiden Körpern beschickt werden.

Derart kultiviert, ist die Differenzierungsfähigkeit der Zellen in den organoide Körper aktiviert und die Zellen können sich insbesondere in Zellen des Ektoderms differenzieren. Die Zellen können sowohl als organoide Körper als auch als einzelne Zellen gelagert und kultiviert werden und behalten ihre Pluripotenz.

### (II) Differenzierung in den organoiden Körpern und/oder Gewebekörpern

Die Differenzierung von Zellen in organoiden Körpern oder Gewebekörpern kann allgemein in einem Kultivierungsmedium mit einem die Differenzierung beeinflussenden Zusatz oder an der Grenze eines Kultivierungsmediums zur gas- oder dampfförmigen Umgebung erfolgen. Die folgenden Beispiele 5 und 6 beschreiben Arbeitsprotokolle für beide Varianten.

### BEISPIEL 5

Für die Induktion der Differenzierung wurden vorzugsweise Stammzellen nach dem 42. Tag der Kultivierung verwendet. Die Verwendung von Stammzellen nach der 3. oder 4. Passage oder von Zellen, die bei der Temperatur von flüssigem Stickstoff 12-18 Monate lang gelagert worden waren, war ebenfalls problemlos möglich.

Zunächst wurden die Zellen in Differenzierungsmedium mit der oben angegebenen Zusammensetzung überführt und auf eine Dichte von etwa 3 x 10⁴ Zellen/ml eingestellt; z.B. durch Trypsinbehandlung einer Stammzelkultur in Nährmedium, 5-minütige Zentrifugation bei 1000 UpM und Resuspendieren des Pellets in Differenzierungsmedium und Verdünnung soweit erforderlich.

Anschließend wurden mit einer 20-µl Pipette ca. 50 20-µl-Tropfen (600 Zellen/20 µl) auf die Innenseite des Deckels einer bakteriologischen Petrischale gegeben (gestopfte Spitzen) und die Deckel vorsichtig auf die mit PBS gefüllten Petrischalen gestülpt, sodass die Tropfen nach unten hängen. Für jeden Deckel wurde eine neue Spitze verwendet. Die Petrischalen wurden anschließend vorsichtig in den Brutschrank gestellt und 48 h lang bei 37°C inkubiert.

Danach wurden die in den hängenden Tropfen aggregierten Zellen, die organoide Körper, aus jeweils vier Deckeln in je eine bakteriologische Petrischale mit 5 ml Inkubationsmedium mit 20 % FKS überführt (Deckel schräg halten und die organoiden Körper mit etwa 2,5 ml Nährmedium abspülen) und für weitere 5-9 Tage, vorzugsweise 96 h, kultiviert.

Die organoiden Körper wurden nun vorsichtig mit einer Pipette aufgesammelt und in mit 0,1 % Gelatine beschichtete Zellkulturgefäße mit Differenzierungsmedium überführt. Die organoiden Körper vermehrten sich nun und wuchsen in zum Teil einzelnen Zellkolonien, die wieder vermehrt vereinzelt und vermehrt werden konnten. In einer besonders bevorzugten Ausgestaltung des Verfahrens wurden als Kulturgefäß mit 0,1 % Gelatine beschichtete 6 cm Petrischalen verwendet, in die 4 ml Differenzierungsmedium vorgelegt worden war, und diese mit je 6 organoiden Körpern beschickt. Ein weiteres bevorzugtes Kulturgefäß waren mit 0,1 % Gelatine beschichtete Chamber Slides, in die 3 ml Differenzierungsmedium vorgelegt wurde und die anschließend mit je 3-8 organoiden Körpern beschickt wurden und Thermanox-Platten (Nalge Nonc International, USA) für elektronenmikroskopische Studien. Ein weitere Alternative waren 24-Mulden-Mikrotiterplatten, die mit 0,1 % Gelatine beschichtet wurden und in die je 1,5 ml pro Mulde Differenzierungsmedium vorgelegt wurde und die anschließend mit je 4 organoiden Körpern beschickt wurden.

In einer bevorzugten Ausgestaltung des Verfahrens wurden organoide Körper etwa 7 Wochen lang in den gelatinebeschichteten 6-cm-Petrischalen kultiviert und danach wurden einzelne organoide Körper mit dem Microdissector (Eppendorf, Hamburg, Deutschland nach den Anweisungen des Herstellers ausgeschnitten und dann z.B. auf frische 6-cm-Petrischalen, Chamber Slides oder Thermanox-Platten überführt.

### BEISPIEL 6

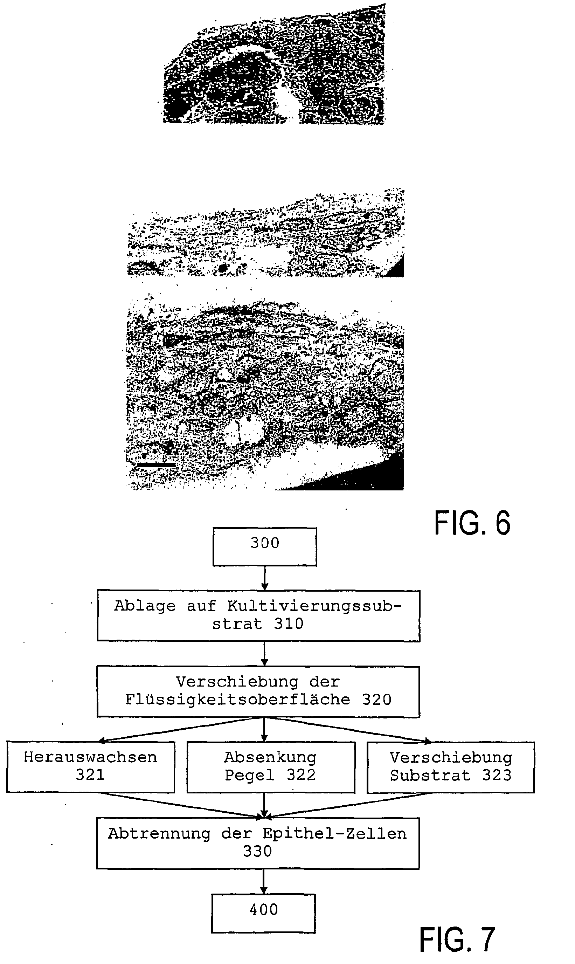

Figur 5 zeigt beispielhaft einen in der Adhäsionskultur wachsenden Gewebekörper 5 (der weiße Strich entspricht einer Länge von 2 mm im Original), wobei ein Teil des Gewebekörpers aus dem Kultivierungsmedium herausragt. Der Gewebekörper 5 ist aus einem organoiden Körper gewachsen, der entsprechend einem der o.g. Verfahren gebildet wurde. In dem gestrichelt markierten Teil des Gewebekörpers 5 werden an der Außenluft Epithelzellen 6 gebildet. Es entstehen Plattenepithelien bis zu einer Größe von mehreren Millimetern. Figur 6 illustriert licht- und elektronenmikroskopische Darstellungen der differenzierten Epithelien (der schwarze Strich im untersten Bild entspricht einer Länge von rd. 7 µm im Original).

In Figur 7 sind weitere Einzelheiten der erfindungsgemäßen Verfahrensweise bei der Bildung der Epithelzellen an der Flüssig-Fest-Grenzfläche illustriert. Die nach dem oben beschriebenen Verfahren erzeugten organoiden Körper oder Gewebekörper werden bei Schritt 310 auf einem Kultivierungssubstrat abgelegt. Anschließend folgt die Verschiebung der Flüssigkeitsoberfläche des Kultivierungsmediums (Schritt 320), bis die Oberseite des organoiden Körpers oder Gewebekörpers die Flüssigkeitsoberfläche berührt. Die weitere Differenzierung der Zellen erfolgt auf mindestens einem der drei illustrierten Wege, nämlich durch ein Herauswachsen (Schritt 321) aus dem Kultivierungsmedium, durch eine Absenkung des Pegels des Kultivierungsmediums (Schritt 322) und/oder eine Verschiebung des Kultivierungssubstrats (Schritt 323). Anschließend werden die gebildeten Epithelzellen abgetrennt (Schritt 330) und einer weiteren Vermehrung zugeführt (siehe Schritt 400 in Figur 1).

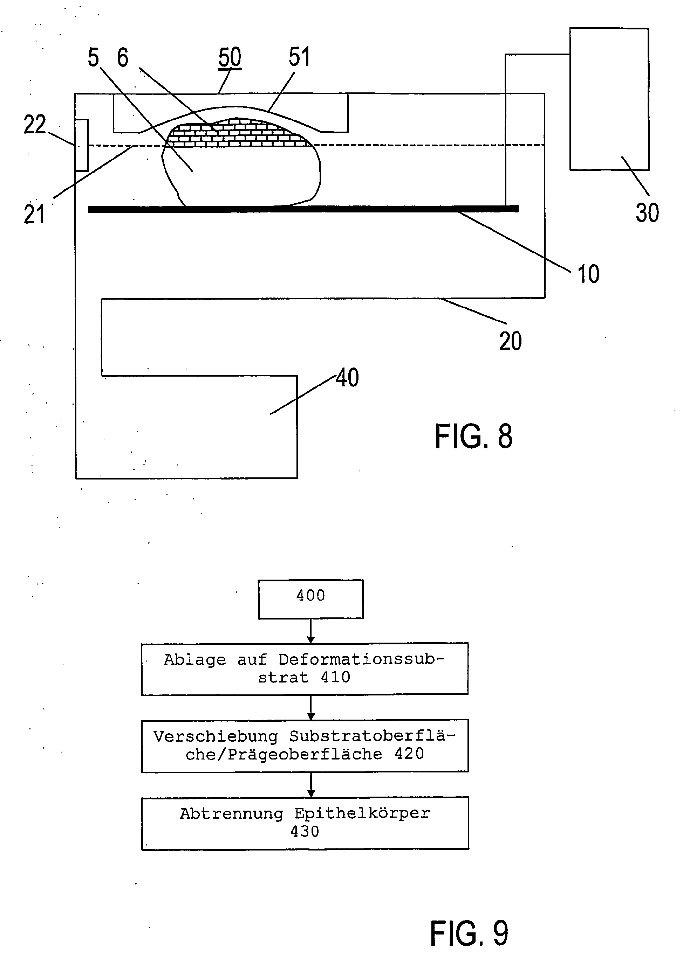

Die Figur 8 zeigt schematisch wichtige Komponenten einer erfindungsgemäßen Kultivierungseinrichtung mit einem Kultivierungssubstrat 10, das im Kultivierungsgefäß 20 verschiebbar und in bestimmten Positionen fixierbar angeordnet ist. Hierzu ist als Steuereinrichtung eine Stelleinrichtung 30 vorgesehen, mit der das Kultivierungssubstrat 10 z. B. mit einem piezoelektrischen Stellglied positionierbar ist. Des Weiteren kann im Kultivierungsgefäß 20 mit der Pegelsteuerung 40 der Füllstand 21 der Kultivierungsflüssigkeit eingestellt werden, die an sich bekannte fluidische Komponenten, wie z. B. Ventile und eine Pumpe enthält. Zur Überwachung des Füllstandes 21 der Kultivierungsflüssigkeit im Kultivierungsgefäß 20 ist ein Sensor 22 vorgesehen. Der Sensor 22 ist bspw. ein optischer Sensor oder ein Leitfähigkeitssensor, mit dem der Füllstand in an sich bekannter Weise detektiert werden kann. Das Sensorsignal kann zur Betätigung der Stelleinrichtung 30 und/oder der Pegelsteuerung 40 im Rahmen eines Regelkreises verwendet werden.

Oberhalb der Flüssigkeitsoberfläche 21 befindet sich ein Prägewerkzeug 50 mit einer Prägeoberfläche 51, mit der den Epithelzellen, die sich auf der Oberfläche des Gewebekörpers 5 bilden, eine bestimmte Form aufgeprägt werden kann.

Der Betrieb der Kultivierungseinrichtung erfolgt entsprechend dem Schema in Figur 7 derart, dass zunächst ein oder mehrere Gewebekörper 5 auf dem Kultivierungssubstrat 10 abgelegt werden. Anschließend erfolgt ein Herauswachsen des Gewebekörpers 5, eine Verschiebung des Kultivierungssubstrats 10 und/oder eine Verstellung des Füllstandes 21, bis ein Teil des Gewebekörpers 5 aus dem Kultivierungsmedium herausragt und zu Epithelzellen differenziert.

Die Verwendung des Prägewerkzeugs 50 gemäß Figur 8 ist nicht zwingend erforderlich. Die Formgebung des Epithelgewebes kann nachfolgend bei der weiteren Vermehrung der differenzierten Epithelzellen (Schritt 400, siehe Figur 1) erfolgen. Typische Verfahrensschritte der weiteren Vermehrung sind in Figur 9 zusammengestellt.

Zunächst erfolgt die Ablage der differenzierten Epithelzellen auf einem Deformationssubstrat (Schritt 410). Anschließend erfolgt die Verschiebung der Substratoberfläche (Schritt 420), so dass die Epithelzellen eine gewünschte Form bilden. Diese Verschiebung kann mit einer zusätzlichen Formgebung mit einem Prägewerkzeug kombiniert werden. Schließlich erfolgt die Abtrennung des Epithelkörpers (Schritt 430).

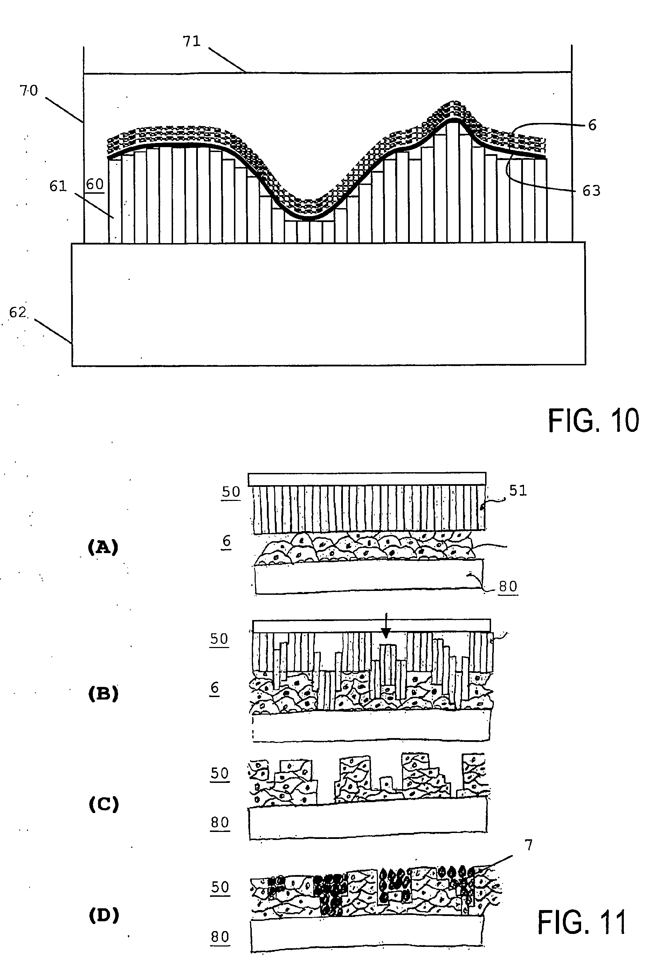

Das Deformationssubstrat, dessen Einzelheiten unter Bezug auf die Figuren 10 und 11 beschrieben werden, dient der Aufnahme und dem Wachstum von Epithelzellen sowie deren Formung. Es kann selbst eine deformierbare Oberfläche besitzen und/oder als Träger der Epithelzellen bei der Einwirkung eines Prägewerkzeuges dienen.

Die Figur 10 illustrier ein Deformationssubstrat 60, das eine Vielzahl von Formelementen 61 aufweist, die jeweils einzeln mit einer Stelleinrichtung 62 verschiebbar sind, die an einem. Basisteil (nicht dargestellt) angebracht ist. Jedes Formelement 61 besitzt eine Quaderform mit einer Oberseite. Die Gesamtzahl der Oberseiten oder mindestens ein auf diesen angeordnetes, nachgiebiges, schichtförmiges Abdeckelement 63 bilden die Oberfläche des Deformationssubstrats 60, die zur Formgebung an der Epithelzusammensetzung 6 verwendet wird. Die Oberseiten der Formelemente 61 besitzen typische Dimensionen im Bereich von 0.01 mm bis 5 mm. Die Formelemente 61 sind so ausgerichtet, dass die Oberseiten eine Matrixanordnung aus geraden Reihen und Spalten bilden. Die Verschiebung der Formelemente 61 relativ zum Basisteil erfolgt bspw. mit Stellmotoren oder piezoelektrischen Antrieben. Je nach dem gewählten Vorschub eines Formelements 61 wird die Oberfläche strukturiert.

Das Abdeckelement 63 besitzt die Vorteile, dass die Werkzeugoberfläche lokal geglättet und die Abnahme des Zellmaterials 6 vom Werkzeug erleichtert wird. Das Abdeckelement 63 umfasst zum Beispiel eine Folie (zum Beispiel Polyurethan) oder eine Membran, die sich über alle Formelemente 61 erstreckt und auf der die Zellen angeordnet sind. Es können alternativ ein oder mehrere Abdeckelemente vorgesehen sein, die sich nur über eine oder mehrere Teilgruppen von Formelementen erstrecken. Es kann ein lösbares Haften des Abdeckelements 63 an einigen oder allen Oberseiten der Formelemente 61 vorgesehen sein.

Das Abdeckelement 63 kann aus einem synthetischen Polymermaterial und/oder aus einem in biologischen Organismen natürlich vorkommenden Material, wie zum Beispiel Chitin oder Knochenmatrixmaterial, in einer oder mehreren Schichten bestehen. Das Abdeckelement 63 kann ferner eine strukturierte Beschichtung tragen, die ein adhärentes Anhaften von biologischen Zellen in Teilbereichen einerseits fördert und in anderen Teilbereichen andererseits blockiert.

Zur erfindungsgemäßen Formung von Epithelzellmaterial 6 wird dieses zunächst auf der Oberfläche des Deformationssubstrates 60, also der Gesamtheit der Oberseiten oder dem gemeinsamen Abdeckelement 63 angeordnet. Hierzu ist bspw. eine Ablage einer Vielzahl von organoiden Körpern oder Gewebekörpern in einem Kultivierungsmedium 71 in einem Kulturgefäß 70 vorgesehen. Anschließend erfolgt ein Wachstum der organoiden Körpern oder Gewebekörper und eine Verstellung der Oberfläche des Deformationssubstrates 60 durch den Vorschub der Formelemente 61 in die jeweils gewünschten Positionen. Dieser Vorschub erfolgt mit der o. g. molekularen Bindungsgeschwindigkeit der Zellen, so dass bei der Deformation des Zellmaterials eine verletzungsfreie Verdrängung und Umordnung der Zellen erfolgen kann. Anschließend kann eine Ablösung des Zellmaterials als Epithelkörper vom Deformationssubstrat 60 erfolgen.

Ein Deformationssubstrat gemäß Figur 10 kann alternativ als Prägewerkzeug 50 verwendet werden, wie dies in der Bildfolge gemäß Figur 11 illustriert ist. In einer Ausgangssituation gemäß Teilbild A befindet sich auf einem Träger 80 ein Epithelzellmaterial 6, das deformiert werden soll. Über der zunächst freien Oberfläche des Zellmaterials 6 wird das Prägewerkzeug 50 mit der Vielzahl von verschiebbaren Formelementen 51 angeordnet. Das Prägewerkzeug 50 wird an das Zellmaterial 6 angenähert, bis die in diesem Fall nach unten weisenden Oberseiten das Zellmaterial 6 berühren. Anschließend erfolgt gemäß Teilbild B eine Verstellung der Prägeoberfläche des Prägewerkzeugs 50 durch den gezielten Vorschub einzelner Formelemente 51. Die Vorschubbewegung erfolgt mit der o. g. molekularen Bindungsgeschwindigkeit biologischer Zellen. Die einzelnen Formelemente 51 verdrängen die Zellen im Zellmaterial verletzungsfrei.

Anschließend wird gemäß Teilbild C das Prägewerkzeug 50 entfernt. Die Oberflächenform des Werkzeugs bleibt als komplementäre Struktur im Zellmaterial 6 bestehen. Für eine Erleichterung der Abtrennung des Prägewerkzeugs 50 vom Zellmaterial 6 können die Oberseiten der Formelemente 51 mit einer Beschichtung versehen sein, auf der ein Anhaften von Zellen unterbunden wird. Die Beschichtung erfolgt bspw. mit dem Polymer Polyhema. Schließlich können die im Zellmaterial eingeprägten Lücken gemäß Teilbild D mit anderen Zellen 7 oder einem synthetischen Matrixmaterial befüllt werden.

Die Auswahl der Form und ggf. in das Zellmaterial zugeführten Zellen oder Zusatzstoffe 7 erfolgt je nach der konkreten Aufgabe im Rahmen des Tissue Engineering. Mit der Abfolge gemäß Figur 11 können bspw. Epithel-Zellen mit einer vorbestimmten Struktur mit Gewebe-Zellen in Verbindung gebracht werden.

Die in der vorstehenden Beschreibung, den Ansprüchen und den Zeichnungen offenbarten Merkmale der Erfindung können sowohl einzeln oder auch in Kombination für die Verwirklichung der Erfindung in ihren verschiedenen Ausgestaltungen von Bedeutung sein.

## Patentansprüche

1. Verfahren zur Bildung von Epithelzellen in Kultur, umfassend:
- Aggregation von adulten Stammzellen aus exokrinem differenzierten Drüsengewebe zu einem organoiden Körper, und
- Differenzieren zumindest eines Teils des organoiden Körpers oder eines daraus gewachsenen Gewebekörpers zu Epithelzellen, wobei das Differenzieren des organoiden Körpers oder des Gewebekörpers ein Kultivieren an einer Grenzfläche zwischen flüssigen und gasförmigen Medien umfasst.

2. Verfahren nach Anspruch 1, bei dem beim Kultivieren an der Grenzfläche eine Flüssigkeitsoberfläche eines Kultivierungsmediums relativ zur Oberfläche des organoiden Körpers oder des Gewebekörpers verschoben wird.

3. Verfahren nach Anspruch 2, bei dem das Verschieben der Flüssigkeitsoberfläche gleichzeitig oder aufeinanderfolgend einen oder mehrere der folgenden Schritte umfasst:
- Herauswachsen (321) des organoiden Körpers oder des Gewebekörpers aus dem Kultivierungsmedium,
- Absenken (322) der Flüssigkeitsoberfläche des Kultivierungsmediums, und
- Anheben (323) eines Kultivierungssubstrats mit dem organoiden Körper oder dem Gewebekörper aus dem Kultivierungsmedium.

4. Verfahren nach Anspruch 2 oder 3, bei dem das Verschieben der Flüssigkeitsoberfläche mit einer Geschwindigkeit relativ zur Oberfläche des organoiden Körpers oder des Gewebekörpers erfolgt, die an eine Umwandlungsgeschwindigkeit beim Differenzieren der Zellen des organoiden Körpers oder des Gewebekörpers zu Epithelzellen angepasst ist.

5. Verfahren nach Anspruch 2 oder 3, bei dem das Verschieben der Flüssigkeitsoberfläche mit einer Geschwindigkeit relativ zur Oberfläche des organoiden Körpers oder des Gewebekörpers erfolgt, die kleiner oder gleich einer molekularen Bindungsgeschwindigkeit der Zellen des organoiden Körpers oder des Gewebekörpers ist.

6. Verfahren nach Anspruch 4 oder 5, bei dem die Geschwindigkeit kleiner oder gleich 1000 µm/h ist.

7. Verfahren nach mindestens einem der vorhergehenden Ansprüche, bei dem das Differenzieren des organoiden Körpers oder des Gewebekörpers in unmittelbarer Nachbarschaft an eine Prägeoberfläche erfolgt, so dass den Epithelzellen eine geometrische Anordnung entsprechend der Form der Prägeoberfläche aufgeprägt wird.

8. Verfahren nach mindestens einem der vorhergehenden Ansprüche, bei dem die Aggregation eine Verbindung von primär aus dem exokrinen Gewebe isolierten Stammzellen zu primären organoiden Körpern oder eine Verbindung von Stammzellen, die aus den primären organoiden Körpern stammen, zu sekundären organoiden Körpern umfasst.

9. Verfahren nach mindestens einem der vorhergehenden Ansprüche, bei dem die differenzierten Epithelzellen vom organoiden Körper oder vom Gewebekörper getrennt und vermehrt werden.

10. Verfahren nach Anspruch 9, bei dem die Vermehrung auf einem flüssigkeitsdurchlässigen Kultivierungssubstrat erfolgt.

11. Verfahren nach Anspruch 10, bei dem die Vermehrung auf einem Kollagenvlies oder einem textilen Gewebe erfolgt.

12. Verfahren nach mindestens einem der vorhergehenden Ansprüche 9 bis 11, bei dem die Vermehrung auf einer fibrinhaltigen Oberfläche erfolgt.

13. Verfahren nach mindestens einem der vorhergehenden Ansprüche 9 bis 11, bei dem die Vermehrung auf einer chitinhaltigen Oberfläche erfolgt.

14. Verfahren nach mindestens einem der vorhergehenden Ansprüche 9 bis 13, bei dem die Vermehrung der Epithelzellen auf einem deformierbaren Kultivierungssubstrat (60) erfolgt.

15. Verfahren nach mindestens einem der vorhergehenden Ansprüche 9 bis 14, wobei die Kultur der vermehrten Epithelzellen einer Deformation mit einer Stempeleinrichtung (50) unterzogen wird.

16. Verfahren nach mindestens einem der vorhergehenden Ansprüche, bei dem die Stammzellen aus einer Drüse eines Wirbeltieres isoliert werden.

17. Verfahren nach Anspruch 16, bei dem die Stammzellen aus einer Drüse eines Fisches, eines Vogels oder eines Säugetieres isoliert werden.

18. Verfahren nach Anspruch 17, bei dem die Stammzellen aus eines juvenilen oder adulten Menschen isoliert werden.

19. Verfahren nach mindestens einem der vorhergehenden Ansprüche 2 bis 18, bei dem beim Kultivieren an der Grenzfläche die Flüssigkeitsoberfläche des Kultivierungsmediums relativ zur Oberfläche des organoiden Körpers oder des Gewebekörpers wiederholt mit einer Richtungsumkehr verschoben wird, so dass der organoide Körper oder der Gewebekörper wiederholt im Kultivierungsmedium untertaucht und wieder auftaucht.

20. Verfahren nach Anspruch 19, bei dem eine periodische Bewegung der Flüssigkeitsoberfläche des Kultivierungsmediums relativ zur Oberfläche des organoiden Körpers oder des Gewebekörpers vorgesehen ist.

21. Kultivierungseinrichtung zur Bildung von differenzierten Epithelzellen nach Anspruch 1, die umfasst:
- ein Kultivierungssubstrat (10) auf dem sich mindestens ein durch Aggregation von adulten Stammzellen aus exokrinem differenzierten Drüsengewebe entstandener organoider Körper oder Gewebekörper befindet,
- ein Kultivierungsgefäß (20) zur Aufnahme eines flüssigen Kultivierungsmediums,
- eine Steuereinrichtung (30, 40) zur Einstellung der Flüssigkeitsoberfläche der Kultivierungsflüssigkeit im Kultivierungsgefäß relativ zur Oberfläche des Kultivierungssubstrats (10), sowie
- ein Prägewerkzeug (50) zur Formgebung am differenzierten Epithelgewebe.

22. Kultivierungseinrichtung zur Bildung von differenzierten Epithelzellen nach Anspruch 1, die umfasst:
- ein deformierbares Kultivierungssubstrat (60) mit einer Vielzahl von einzeln verschiebbaren Formelementen (61) auf dem sich mindestens ein durch Aggregation von adulten Stammzellen aus exokrinem differenzierten Drüsengewebe entstandener organoider Körper oder Gewebekörper befindet,
- ein Kultivierungsgefäß (20) zur Aufnahme eines flüssigen Kultivierungsmediums, und
- eine Steuereinrichtung (30, 40) zur Einstellung der Flüssigkeitsoberfläche der Kultivierungsflüssigkeit im Kultivierungsgefäß relativ zur Oberfläche des Kultivierungssubstrats (10).

23. Kultivierungseinrichtung nach Anspruch 21 oder 22, bei der die Steuereinrichtung eine Stelleinrichtung (30) zur Positionierung und/oder Verschiebung des Kultivierungssubstrats (10) in einer vorbestimmten Höhe im Kultivierungsgefäß (20) umfasst.

24. Kultivierungseinrichtung nach Anspruch 21 oder 22, bei der die Steuereinrichtung eine Pegelsteuerung (40) zur Einstellung eines Füllstandes der Kultivierungsflüssigkeit im Kultivierungsgefäß (20) umfasst.

25. Kultivierungseinrichtung einem der Ansprüche 21 bis 24, bei der die Steuereinrichtung dazu eingerichtet ist, die Flüssigkeitsoberfläche der Kultivierungsflüssigkeit im Kultivierungsgefäß relativ zur Oberfläche des Kultivierungssubstrats (10) wiederholt mit einer Richtungsumkehr zu verschieben.

26. Kultivierungseinrichtung nach Anspruch 22, bei der ein Prägewerkzeug (50) zur Formgebung am differenzierten Epithelgewebe vorgesehen ist.

## Claims

1. A process for the generation of epithelial cells in culture, comprising:
- an aggregation of adult stem cells from differentiated exocrine glandular tissue to an organoid body, and
- differentiation of at least a part of the organoid body or of a tissue body grown from it to epithelial cells, wherein the differentiation of the organoid body or tissue body comprises a cultivation on a boundary surface between liquid and gaseous media.

2. The process according to Claim 1, in which a liquid surface of a cultivation medium is shifted relative to the surface of the organoid body or tissue body during the cultivation on the boundary surface.

3. The process according to Claim 2, in which the shifting of the liquid surface comprises simultaneously or successively one or more of the following steps:
- growing out (321) of the organoid body or of the tissue body from the cultivation medium,
- lowering (322) of the liquid surface of the cultivation medium, and
- raising (323) of a cultivation substrate with the organoid body or the tissue body from the cultivation medium.

4. The process according to Claim 2 or 3, in which the shifting of the liquid surface takes place at a rate relative to the surface of the organoid body or of the tissue body that is adapted to a conversion rate when differentiating cells of the organoid body or of the tissue body to epithelial cells.

5. The process according to Claim 2 or 3, in which the shifting of the liquid surface takes place at a rate relative to the surface of the organoid body or of the tissue body that is less than or equal to a molecular bonding rate of the cells of the organoid body or of the tissue body.

6. The process according to Claim 4 or 5, in which the rate is less than or equal to 1000 µm/h.

7. The process according to at least one of the preceding claims, in which the differentiation of the organoid body or of the tissue body takes place in the immediate vicinity of an imprinting surface in such a manner that a geometric arrangement corresponding to the form of the imprinting surface is imprinted on the epithelial cells.

8. The process according to at least one of the preceding claims, in which the aggregation comprises an association of stem cells isolated primarily from the exocrine tissue to primary organoid bodies or comprises an association of stem cells derived from the primary organoid bodies to secondary organoid bodies.

9. The process according to at least one of the preceding claims, in which the differentiated epithelial cells are separated from the organoid body or from the tissue body and propagated.

10. The process according to Claim 9, in which the propagation takes place on a liquid-permeable cultivation substrate.

11. The process according to Claim 10, in which the propagation takes place on a collagen fleece or a textile tissue.

12. The process according to at least one of the preceding claims 9 to 11, in which the propagation takes place on a fibrin-containing surface.

13. The process according to at least one of the preceding claims 9 to 11, in which the propagation takes place on a chitin-containing surface.

14. The process according to at least one of the preceding claims 9 to 13, in which the propagation of the epithelial cells takes place on a deformable cultivation substrate (60).

15. The process according to at least one of the preceding claims 9 to 14, in which the culture of the propagated epithelial cells is subjected to a deformation with an imprinting tool (50).

16. The process according to at least one of the preceding claims in which the stem cells are isolated from a gland of a vertebrate.

17. The process according to Claim 16, in which the stem cells are isolated from a gland of a fish, a bird or a mammal.

18. The process according to Claim 17, in which the stem cells are isolated from a juvenile or adult human.

19. The process according to at least one of the preceding claims 2 to 18, in which during the cultivation on the boundary surface the liquid surface of the cultivation medium is repeatedly shifted with a reversal of direction relative to the surface of the organoid body or of the tissue body so that the organoid body or the tissue body is repeatedly submerged in and reappears from the cultivation medium.

20. The process according to Claim 19, in which a periodic movement of the liquid surface of the cultivation medium relative to the surface of the organoid body or of the tissue body is provided.

21. A cultivation device for generating differentiated epithelial cells according to Claim 1, comprising:
- a cultivation substrate (10) on which at least one organoid body or tissue body generated by aggregation of adult stem cells from differentiated exocrine glandular tissue is provided,
- a cultivation vessel (20) for receiving a liquid cultivation medium,
- a controlling means (30, 40) for adjusting the liquid surface of the cultivation liquid in the cultivation vessel relative to the surface of the cultivation substrate (10), and
- an imprinting tool (50) for shaping on the differentiated epithelial tissue.

22. A cultivation device for generating differentiated epithelial cells according to Claim 1, comprising:
- a deformable cultivation substrate (60) with a plurality of forming elements (61) which can be shifted individually, on which at least one organoid body or tissue body generated by aggregation of adult stem cells from differentiated exocrine glandular tissue is provided,
- a cultivation vessel (20) for receiving a liquid cultivation medium, and
- a controlling means (30, 40) for adjusting the liquid surface of the cultivation liquid in the cultivation vessel relative to the surface of the cultivation substrate (10).

23. The cultivation device according to Claim 21 or 22, in which the controlling means comprises a positioning means (30) for positioning and/or shifting the cultivation substrate (10) in a predetermined height in the cultivation vessel (20).

24. The cultivation device according to Claim 21 or 22, in which the controlling means comprises a level control (40) for adjusting a filling level of the cultivation liquid in the cultivation vessel (20).

25. The cultivation device according to any one of Claims 21 to 24, in which the controlling means is designed to repeatedly shift the liquid surface of the cultivation liquid in the cultivation vessel relative to the surface of the cultivation substrate (10) with a reversal of direction.

26. The cultivation device according Claim 22, in which an imprinting tool (50) is provided for shaping on the differentiated epithelial tissue.

## Revendications

1. Procédé de préparation de cellules épithéliales en culture, comportant :
- agrégation de cellules souches adultes du tissu glandulaire exocrine différencié pour former un corps organoïde, et
- différenciation d'au moins une partie du corps organoïde ou d'un corps tissulaire développé à partir de celui-ci pour donner des cellules épithéliales, la différenciation du corps organoïde ou du corps tissulaire comportant une culture sur une interface entre des milieux liquides et gazeux.

2. Procédé selon la revendication 1, dans lequel, pendant la culture sur l'interface, une surface liquide d'un milieu de culture est déplacée par rapport à la surface du corps organoïde ou du corps tissulaire.

3. Procédé selon la revendication 2, dans lequel le déplacement de la surface liquide comporte simultanément ou successivement une ou plusieurs des étapes suivantes :
- croissance (321) du corps organoïde ou du corps tissulaire hors du milieu de culture,
- abaissement (322) de la surface liquide du milieu de culture, et
- rehaussement (323) d'un substrat de culture avec le corps organoïde ou le corps tissulaire hors du milieu de culture.

4. Procédé selon la revendication 2 ou 3, dans lequel le déplacement de la surface liquide s'effectue à une vitesse relative par rapport à la surface du corps organoïde ou du corps tissulaire, laquelle est ajustée à une vitesse de transformation lors de la différenciation des cellules du corps organoïde ou du corps tissulaire pour obtenir des cellules épithéliales.

5. Procédé selon la revendication 2 ou 3, dans lequel le déplacement de la surface liquide s'effectue à une vitesse relative par rapport à la surface du corps organoïde ou du corps tissulaire, laquelle est inférieure ou égale à une vitesse de liaison moléculaire des cellules du corps tissulaire ou du corps tissulaire.

6. Procédé selon la revendication 4 ou 5, dans lequel la vitesse est inférieure ou égale à 1 000 µm/h.

7. Procédé selon au moins l'une des revendications précédentes, dans lequel la différenciation du corps organoïde ou du corps tissulaire s'effectue à proximité immédiate d'une surface estampée, de manière à imprimer aux cellules épithéliales un agencement géométrique correspondant à la forme de la surface estampée.

8. Procédé selon au moins l'une des revendications précédentes, dans lequel l'agrégation comporte une liaison de cellules souches isolées en premier hors du tissu exocrine pour obtenir des corps organoïdes primaires ou une liaison de cellules souches issues des corps organoïdes primaires pour obtenir des corps organoïdes secondaires.

9. Procédé selon au moins l'une des revendications précédentes, dans lequel les cellules épithéliales différenciées sont séparées du corps organoïde ou du corps tissulaire et sont multipliées.

10. Procédé selon la revendication 9, dans lequel la multiplication est effectuée sur un substrat de culture perméable au liquide.

11. Procédé selon la revendication 10, dans lequel la multiplication est effectuée sur un non-tissé collagéneux ou un tissu textile.

12. Procédé selon au moins l'une des revendications précédentes 9 à 11, dans lequel la multiplication est effectuée sur une surface contenant de la fibrine.

13. Procédé selon au moins l'une des revendications précédentes 9 à 11, dans lequel la multiplication est effectuée sur une surface contenant de la chitine.

14. Procédé selon au moins l'une des revendications précédentes 9 à 13, dans lequel la multiplication des cellules épithéliales est effectuée sur un substrat de culture (60) déformable.

15. Procédé selon au moins l'une des revendications précédentes 9 à 14, dans lequel la culture des cellules épithéliales multipliées est soumise à une déformation au moyen d'un dispositif d'estampage (50).

16. Procédé selon au moins l'une des revendications précédentes, dans lequel les cellules souches sont isolées hors d'une glande d'un vertébré.

17. Procédé selon la revendication 16, dans lequel les cellules souches sont isolées hors d'une glande d'un poisson, d'un oiseau ou d'un mammifère.

18. Procédé selon la revendication 17, dans lequel les cellules souches sont isolées hors d'un homme jeune ou adulte.

19. Procédé selon au moins l'une des revendications précédentes 2 à 18, dans lequel, lors de la culture sur l'interface, la surface liquide du milieu de culture est déplacée par rapport à la surface du corps organoïde ou du corps tissulaire de manière répétée avec une inversion du sens de déplacement, de telle sorte que, de manière répétée, le corps organoïde ou le corps tissulaire s'immerge dans le milieu de culture et en émerge à nouveau.

20. Procédé selon la revendication 19, **caractérisé en ce qu'**il est prévu un déplacement périodique de la surface liquide du milieu de culture par rapport à la surface du corps organoïde ou du corps tissulaire.

21. Dispositif de culture pour la préparation de cellules épithéliales différenciées selon la revendication 1, lequel comporte :
- un substrat de culture (10), sur lequel se situe au moins un corps organoïde ou un corps tissulaire obtenu par l'agrégation de cellules souches adultes d'un tissu glandulaire exocrine différencié,
- un récipient de culture (20) destiné à recevoir un milieu de culture liquide,
- un dispositif de commande (30, 40) destiné à régler la surface liquide du liquide de culture dans le récipient de culture par rapport à la surface du substrat de culture (10), ainsi que
- un outil d'estampage (50) pour le formage sur le tissu épithélial différencié.

22. Dispositif de culture pour la préparation de cellules épithéliales différenciées selon la revendication 1, lequel comporte :
- un substrat de culture (60) déformable avec une pluralité d'éléments profilés (61) mobiles séparément, sur lequel se situe au moins un corps organoïde ou un corps tissulaire obtenu par l'agrégation de cellules souches adultes d'un tissu glandulaire exocrine différencié,
- un récipient de culture (20) destiné à recevoir un milieu de culture liquide,
- un dispositif de commande (30, 40) destiné à régler la surface liquide du liquide de culture dans le récipient de culture par rapport à la surface du substrat de culture (10).

23. Dispositif de culture selon la revendication 21 ou 22, dans lequel le dispositif de commande comporte un dispositif de réglage (30) pour le positionnement et/ou le déplacement du substrat de culture (10) dans une hauteur prédéterminée dans un récipient de culture (20).

24. Dispositif de culture selon la revendication 21 ou 22, dans lequel le dispositif de commande comporte une commande de niveau (40) pour le réglage d'un niveau du liquide de culture dans le récipient de culture (20).

25. Dispositif de culture selon l'une quelconque des revendications 21 à 24, dans lequel le dispositif de commande est conçu pour déplacer de manière répétée avec une inversion du sens de déplacement la surface liquide du liquide de culture par rapport à la surface du substrat de culture (10).

26. Dispositif de culture selon la revendication 22, dans lequel il est prévu un outil d'estampage (50) pour le formage sur le tissu épithélial différencié.
